# EUROPEAN PATENT APPLICATION

(11) **EP 2 116 481 A1**
(43) Date of publication of application: **11.11.2009**
(21) Application number: 08704051.5
(22) Date of filing: 29.01.2008
(51) Int. Cl.: B65D 81/24, A23L 1/00, A23L 1/025, A23L 3/00, A23L 3/015, A61L 2/02, A61L 2/26, B65D 77/22, G01L 11/00

(54) **PACKAGE FOR HIGH-PRESSURE TREATMENT AND HIGH-PRESSURE TREATMENT METHOD FOR FOODS**

(30) Priority: 05.03.2007 JP 2007054467
(71) Applicant: Kabushiki Kaisha Kobe Seiko Sho, Chuo-ku Kobe-shi Hyogo 651-8585 (JP)
(72) Inventor: The designation of the inventor has not yet been filed
(74) Representative: TBK-Patent
(86) International application number: PCT/JP2008/051242
(87) International publication number: WO 2008/108120

(57) **Abstract**

It is an object of the present invention to provide a package for high pressure treatment of foods and a method for treating foods at a high pressure, both being able to easily determine whether the foods accommodated in the package have been subjected to a high pressure treatment or not. To this end, the package for high pressure treatment includes a package body 7 for accommodating articles to be treated at a high pressure, and an irreversible pressure-sensitive marker 1 disposed on the package body 7 at a position capable of being seen from the exterior of the package body 7 and adapted to develop color when pressurized.

## Description

### [Field of Art]

The present invention relates to a package to be used for treating foods or the like under a specific high pressure condition. Particularly, the present invention is concerned with a package for high pressure treatment capable of being easily determined whether it has been subjected to a high pressure treatment or not, as well as a method for treating foods at a high pressure.

### [Background Art]

Recently, in the field of packaged food, a technique applying a high pressure treatment of several hundred MPa to minimizing the heat denaturation of food components for improving the added value of food typified by flavor and the like attracts attention as a technique in substitution for the conventional sterilization by heating or as an improved treatment technique from the standpoint of the tendency to heighten the added value of food and the improvement of the shelf life.

The above high pressure treatment is sometimes used in combination with heat treatment. For example, in the case of a low acid food, it is necessary to exterminate the spore-forming bacteria. However it has become known that only the above high pressure treatment is not sufficient to exterminate the spore strain even at a pressure as high as 600 MPa. Therefore, the high pressure treatment is used in combination with heat treatment.

The above high pressure treatment and heat treatment are performed with foods accommodated in a container. For example, foods are contained in a flanged container composed of polypropylene resin or PET (polyethylene terephthalate) resin having been subjected to deep drawing. Liquid such as water is added to smooth the transfer of pressure in high pressure treatment, and a film (top film) for closing an opening is applied to an upper surface of the container and is heat-sealed at a flanged edge portion of the container.

The foods thus sealed within the container are placed into a high pressure apparatus which uses water or warm water as pressurizing medium and are then treated at a predetermined pressure for a predetermined time. At this time, since the liquid is pressurized adiabatically, exothermic phenomenon occurs due to adiabatic compression. For example, applied pressures of 400 MPa and 800 MPa accompany temperature rises of about 6°C and about 18°C respectively, and the foods accommodated in the container undergo an isotropic compression together with the container. Upon pressure reduction after the pressurizing treatment, the temperature returns to the original room temperature and the container which has been contracted under compression also reverts to its original dimensions.

However, if a large number of packaged foods are subjected to the high pressure treatment in such a form, there arises the problem that it is practically impossible to make distinction between containers having been subjected to the high pressure treatment and those not having been subjected to the same treatment. As will be apparent from the above description on the process, after the containers are subjected to a high pressure treatment using water or warm water, the containers often do not have any significant change in appearance. Thus, there remains no evidence of having gone through the high pressure treatment, making it very difficult to make distinction from those before the treatment.

In case of performing a heat treatment subsequent to the high pressure treatment and in case of treating products on a production line on which the products flow in one direction, it is possible to distinguish whether a product has been subjected to the high pressure treatment or not if the minute process control is performed. However, in a working environment in which there are various kinds of products in a mixed state, it is impossible to make the aforesaid distinction, which is a serious problem in the production.

Focusing on the above-mentioned problem, such a heat treatment checking system in a heat treatment process for food as is described in Patent Literature 1 is proposed heretofore. Fig. 5 is a perspective view showing a part of the heat treatment checking system and an outline of a retort food sterilizing process.

This heat treatment checking system 100 includes a mount (a product accommodating basket) 30 for conveying a to-be-treated material (retort food A) to an apparatus for performing heating or cooling treatment for food, a detection sensor 10B provided in the mount 30 and capable of storing the history of treatment temperature, and a deformation checking sensor 20. The detection sensor 10B is a shape memory alloy adapted to change in shape depending on the heat treatment temperature and the deformation checking sensor 20 detects a state of deformation of the shape memory alloy directly or indirectly.

However, because the detection sensor 10B capable of storing the history of treatment temperature is provided for each mount 30 which conveys a to-be-treated material, the conventional heat treatment checking system 100 does not determines whether each individual to-be-treated material accommodated in the mount 30 has been heat-treated or not. Thus, it is unfortunately impossible to track each individual to-be-treated material taken out from the mount 30 in the conventional system. Further, in the heat treatment checking system 100, the history of treatment temperature can be stored for each mount 30 for conveying the to-be-treated material, but it is impossible to store the history of pressurizing treatment.
[Patent Literature 1] Japanese Patent Laid Open No. 2002-142736

### [Disclosure of the Invention]

It is an object of the present invention to provide a package for high pressure treatment and a method for treating foods at a high pressure, both for solving the above-mentioned problems of the related art and being able to easily determine whether foods accommodated in the package have been subjected to a high pressure treatment or not.

For achieving the above-mentioned object, the package for high pressure treatment according to the present invention comprises a package body for accommodating articles to be subjected to a high pressure treatment and an irreversible pressure-sensitive marker disposed on the package body at a position capable of being seen from the exterior of the package body and adapted to develop color upon being pressurized. The method for treating foods at a high pressure according to the present invention comprises accommodating foods within the package body of the package for high pressure treatment and subjecting the package for high pressure treatment to a high pressure treatment in the accommodated state of the foods.

### [Brief Description of the Drawings]

Fig. 1 is a perspective view showing a high pressure treatment package for foods according to a first embodiment of the present invention.
Fig. 2 is a schematic sectional view showing a sectional construction of an irreversible pressure-sensitive marker according to the first embodiment.
Fig. 3 is a schematic sectional view showing a sectional state in which the irreversible pressure-sensitive marker according to the first embodiment is destroyed under a pressing force.
Fig. 4 is a perspective view showing an irreversible pressure-sensitive marker according to a second embodiment of the present invention, corresponding to an enlarged perspective view of portion X in Fig. 1.

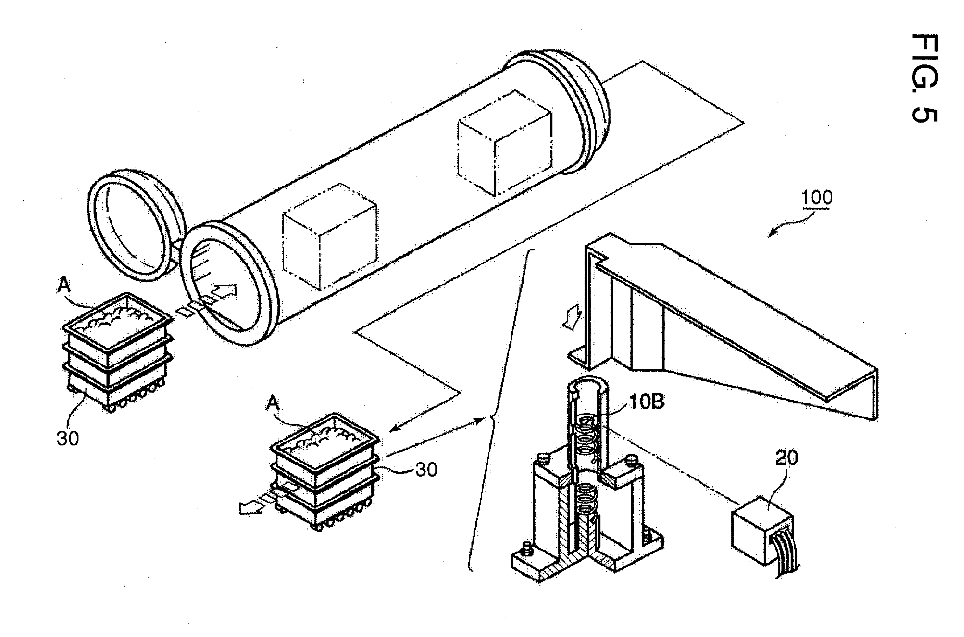

Fig. 5 is a perspective view showing a part of a conventional heat treatment checking system in a food heat-treating process and also showing an outline of a retort food sterilizing process.

### [Best Mode for Carrying Out the Invention]

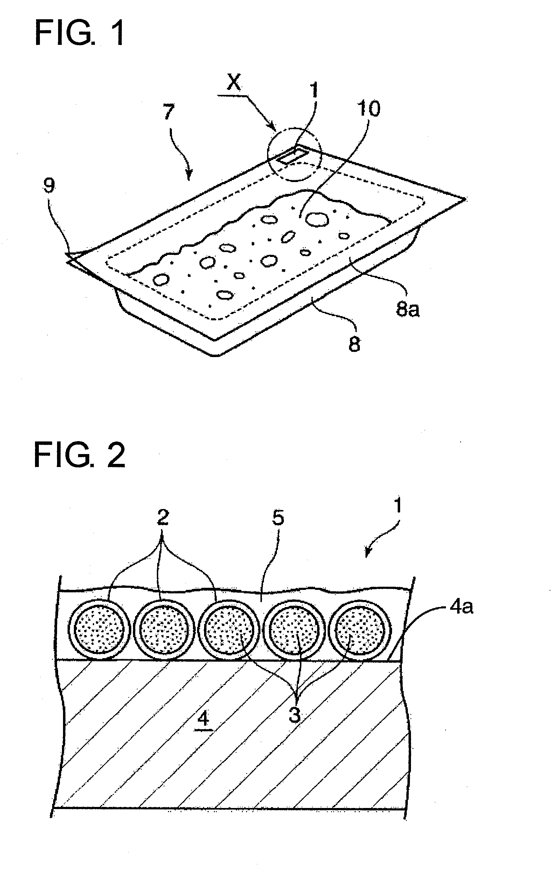

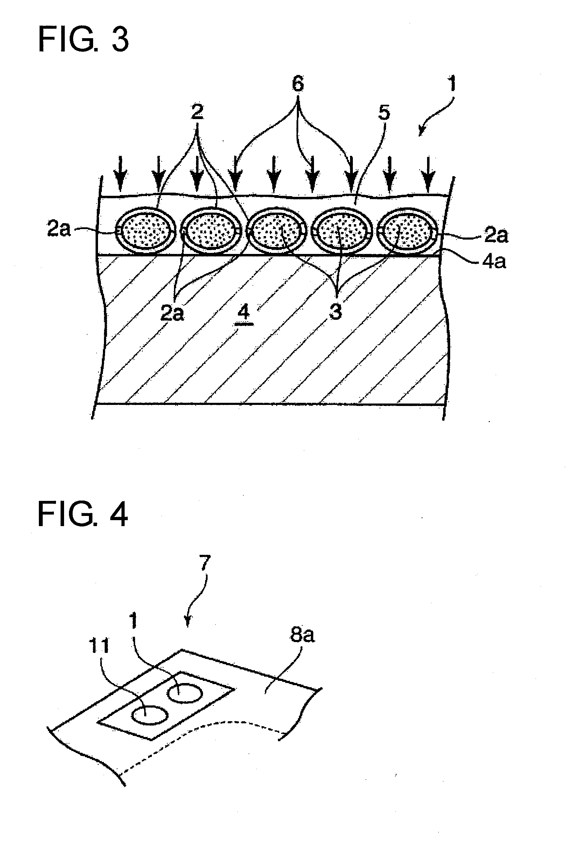

First, a high pressure treatment package for foods according to a first embodiment of the present invention will be described with reference to Figs. 1 to 3. Fig. 1 is a perspective view showing a high pressure treatment package for foods according to a first embodiment of the present invention, Fig. 2 is a schematic sectional view showing a sectional construction of an irreversible pressure-sensitive marker according to the first embodiment, and Fig. 3 is a schematic sectional view showing a sectional state in which the irreversible pressure-sensitive marker according to the first embodiment is destroyed under a pressing force.

The high pressure treatment package for foods according to this first embodiment is for treating foods 10 shown in Fig. 1 at a high pressure and it includes a package body 7 which accommodates the foods 10 in the interior thereof and an irreversible pressure-sensitive marker 1 disposed on (for example, applied) the package body 7 at a position capable of being seen from the exterior of the package body 7 and adapted to develop color upon being pressurized. The package body 7 includes a flanged container 8 and a top film 9. The flanged container 8 is formed of a rigid plastic material, having a thickness of 100 to 300 µm, with a flange portion 8a being formed along edges of an opening. The top film 9 is composed of a soft resin, having a thickness of 50 to 100 µm, and is bonded to the flange portion 8a. The irreversible pressure-sensitive marker 1 is applied on the top film 9 at a position capable of being seen from the exterior of the top film 9.

On the other hand, in the high pressure treatment for foods, isotropic compression which utilizes the pressure of liquid is usually performed as noted above, therefore, an elastic material often tends to undergo a reversible change such that it is deformed and contracted isotropically when pressurized and reverts to its originals state when pressure is reduced. For example, in the case of a spherical shape, the original shape tends to be restored without being destroyed.

Therefore, the marker as shown in Fig. 2 which has such capsules (microcapsules) 2 destroyed by pressure or load and with pigment 3 sealed within each of the capsules, may be used as the irreversible pressure-sensitive marker 1. When pressure or load is applied to the marker 1, the microcapsules 2 with the pigment 3 sealed therein are destroyed and the interior pigment 3 oozes out and colors the surrounding material.

However, even in the case of a microcapsule comprising a hollow spherical shell having a diameter of several ten to several hundred micron meters, with a predetermined reagent or the like sealed within the shell, if the whole of the microcapsule undergoes isotropic compression, the shell is often merely deformed elastically and not destroyed until the pressure reaches a certain level. For destroying the microcapsule surely at a predetermined pressure or higher, it is important that anisotropic pressure be applied thereto.

To meet this condition, the irreversible pressure-sensitive marker 1 according to the first embodiment of the present invention includes a base member 4 having rigidity. The base member 4 has an installation surface, and microcapsules 2 are disposed dispersedly on the installation surface. Also, a transparent film 5 composed of a soft material is formed on the installation surface so as to cover the microcapsules 2. The transparent film 5 comprises a transparent adhesive which is applied after the dispersive arrangement of the microcapsules 2 on the base member 4, or a thin, transparent film which is applied after the dispersive arrangement.

"Having rigidity" means that the base member possesses rigidity high enough to retain its shape against the pressure used in the high pressure treatment for the high pressure treatment package. "Having rigidity" has the same meaning in the following description.

When the high pressure treatment package with the irreversible pressure-sensitive marker 1 disposed thereon is subjected to the high pressure treatment, as shown in Fig. 3, anisotropic pressure, i.e., a vertical pressing force 6, is exerted through the transparent film 5 on the microcapsules 2 arranged on the installation surface 4a of the base member 4 having rigidity, thus making it possible to destroy the spherical microcapsules 2. Cracks 2a are developed in the microcapsules 2 as a result of the destruction and the pigment 3 oozes out from the cracks 2a, presenting a color capable of being recognized visually through the transparent film 5.

As the pigment 3 contained in the microcapsules 2, one which develops color by reaction with another substance (a color former) may be used. In this case, two types of microcapsules 2, that is, microcapsules 2 which contain pigment and microcapsules which contain a color former, may be used. As a simple example, a combination of a dilute iodine solution and starch is taken.

The irreversible pressure-sensitive maker 1 according to the present invention does not always require the dedicated base member 4. For example, in a package printing process for the package body 7, a print material with the pigment-sealed microcapsules 2 added thereto may be printed to a portion of the package body 7 which can be seen from the exterior, thereby the irreversible pressure-sensitive marker 1 can be formed. The print material in this case preferably has a color capable of being distinguished from the color of the pigment which oozes out as a result of destruction of the microcapsules 2, or transparency.

In the case where the package body 7 is constituted by the flanged container 8 composed of a rigid resin material, namely, a resin material having rigidity, as shown in Fig. 1, if the irreversible pressure-sensitive marker 1 is disposed on the flange portion 8a, the flange portion 8a plays the role of the base member having rigidity, therefore, the irreversible pressure-sensitive marker 1 itself is not always required to include the base member 4 composed of a material having rigidity.

However, in the case where the package body 7 is a mere bag composed of a soft resin material, the irreversible pressure-sensitive marker 1 preferably includes the base member 4 composed of a rigid resin in order to exert an anisotropic pressing force on the microcapsules 2.

Next, with reference to Fig. 4, a description will be given about a high pressure treatment package for foods according to a second embodiment of the present invention. Fig. 4 is a partial detailed perspective view showing a principal portion of a package for high pressure treatment according to a second embodiment of the present invention, corresponding to an enlarged view of portion X shown in Fig. 1. This second embodiment is different from the previous first embodiment in that the second embodiment has an irreversible temperature-sensitive marker 11 in addition to the irreversible pressure-sensitive marker 1. Other constructional points are just the same as in the first embodiment. Therefore, the same portions as in the first embodiment are denoted by the same references numerals and the difference will be described below.

In the high pressure treatment package for foods according to the previous first embodiment, the irreversible pressure-sensitive marker 1 adapted to develop color when pressurized is disposed on the package body 7 at a position capable of being seen from the exterior of the package body 7. On the other hand, the high pressure treatment package for foods according to this second embodiment is provided with an irreversible temperature-sensitive marker 11 which develops color at a heat treatment temperature, in addition to the irreversible pressure-sensitive marker 1. As the irreversible temperature-sensitive marker 11, a commercially available thermo-label whose color development level changes depending on the heat treatment temperature may be used.

According to this package for high pressure treatment, in the case where the foods accommodated in the package body 7 are to be subjected to both high pressure treatment and heat treatment, it is possible to easily determine whether the foods go through both the treatments or not from a change in color of the two markers 1 and 11.

As described above, since the package for high pressure treatment according to the present invention includes a package body for accommodating articles to be subjected to the high pressure treatment, and an irreversible pressure-sensitive marker disposed on the package body at a position capable of being seen from the exterior of the package and adapted to develop color when pressurized, it is possible to recognize obviously whether each individual package for high pressure treatment is subjected to the high pressure treatment or not.

The articles to be accommodated in the package for high pressure treatment according to the present invention are not limited to foods. The package is also applicable to, for example, water-containing liquid medical drugs (e.g., instillation) to which heat sterilization or radioactive sterilization is not applicable. It is further applicable to resinous bags for containing chemicals, as well as medical instruments. However, the present invention is effective particularly for high pressure treatment for foods. Recently, the high pressure treatment for foods which improve the shelf life attracts attention, and it is thought that the high pressure treatment will become more and more popular in future. With the high pressure treatment package for foods according to the present invention, it becomes easier to determine whether the foods are subjected to the high pressure treatment or not in process control during manufacture of high pressure-treated foods, or it becomes easier for a seller or a consumer to determine whether the commodity concerned is subjected to the high pressure treatment as indicated thereon at a retail store. Thus, from the standpoint of not only process control but also ensuring the safety of food, the package of the present invention makes a great contribution to the development of the food industry and the soundness of the society.

As the irreversible pressure-sensitive marker, it is preferable to use, for example, a marker comprising a base member having an installation surface, microcapsules disposed dispersedly on the installation surface of the base member and with pigment sealed therein, and a transparent film formed on the installation surface, the microcapsules having a function of being destroyed and developing color upon being pressurized, or a marker comprising a base member, and a print material formed on the base member by printing and containing microcapsules with pigment sealed therein, the microcapsules having a function of being destroyed and developing color upon being pressurized.

Since anisotropic pressure acts on the microcapsules of the irreversible pressure-sensitive marker from the side opposite to the installation surface, the microcapsules are more likely to be destroyed in comparison with the case where isotropic pressure acts on the microcapsules. The pigment oozing out from the destroyed microcapsules colors an appropriate portion to exhibit distinction.

The package body preferably includes a flanged container having a flange portion, and a top film bonded to the flange portion to maintain the articles accommodated within the flanged container in a sealed condition. With respect to the package body, the irreversible pressure-sensitive marker may be disposed on an outer surface of the flanged container or at a bonded portion of the top film.

If the package body includes a portion composed of a rigid material and having an installation surface, and the irreversible pressure-sensitive marker includes microcapsules disposed dispersedly on the installation surface of the package body and with pigment sealed therein and further includes a transparent film formed on the installation surface, and the microcapsules has a function of being destroyed and developing color upon being pressurized, then the irreversible pressure-sensitive marker no longer requires a dedicated base member because the package body plays the role of the base member.

The package body may include a portion composed of a rigid material and having an installation surface, the irreversible pressure-sensitive marker may comprise a print material formed on the installation surface of the package body by printing and containing microcapsules with pigment sealed therein, and the microcapsules may have a function of being destroyed and developing color upon being pressurized.

More specifically, it is preferable that the package body includes a flanged container having a flange portion and also includes a top film bonded to the flange portion to maintain the articles accommodated within the flanged container in a sealed condition, and the flange portion is composed of a rigid material and has the installation surface.

It is more preferable that the package for high pressure treatment of the present invention further comprises an irreversible temperature-sensitive marker provided on the package body and adapted to develop color at a heat treatment temperature. In the case where the articles accommodated within the package body are to be subjected to both high pressure treatment and heat treatment, the irreversible temperature-sensitive marker makes it possible to recognize whether both the treatments are completed for the articles or not.

The present invention also provides a method for treating foods at a high pressure, comprising: accommodating foods within the package body of the package for high pressure treatment, and subjecting the package for high pressure treatment to a high pressure treatment in the accommodated state of the foods.

According to this method, it is possible to easily recognize, after the high pressure treatment, whether the package is actually subjected to the high pressure treatment or not.

Further, in a method according to the present invention, comprising accommodating foods within the package body of the package for high pressure treatment, the package body being provided with the irreversible temperature-sensitive marker, and subjecting the package for high pressure treatment to both high pressure treatment and heat treatment in the accommodated state of the foods, it is also possible to easily recognize whether the package is actually subjected to heat treatment or not in addition to the high pressure treatment.

## Claims

1. A package for high pressure treatment, the package accommodating articles in the interior thereof for subjecting the articles to a high pressure treatment, the package comprising:
a package body accommodating the articles; and
an irreversible pressure-sensitive marker disposed on said package body at a position capable of being seen from the exterior of said package body and adapted to develop color upon being pressurized.

2. The package for high pressure treatment according to claim 1, wherein said irreversible pressure-sensitive marker includes a base member having an installation surface, microcapsules disposed dispersedly on said installation surface of said base member and with pigment sealed therein, and a transparent film formed on said installation surface, said microcapsules having a function of being destroyed and developing color upon being pressurized.

3. The package for high pressure treatment according to claim 1, wherein said irreversible pressure-sensitive marker includes a base member, and a print material formed on said base member by printing and containing microcapsules with pigment sealed therein, said microcapsules having a function of being destroyed and developing color upon being pressurized.

4. The package for high pressure treatment according to claim 1, wherein said package body includes a flanged container having a flange portion, and a top film bonded to said flange portion to maintain the articles accommodated within said flanged container in a sealed condition, and said irreversible pressure-sensitive marker is disposed on an outer surface of said flanged container or at a bonded portion of said top film.

5. The package for high pressure treatment according to claim 1, wherein said package body includes a portion composed of a rigid material and having an installation surface, and said irreversible pressure-sensitive marker includes microcapsules disposed dispersedly on said installation surface of said package body and with pigment sealed therein and also includes a transparent film formed on said installation surface, said microcapsules having a function of being destroyed and developing color upon being pressurized.

6. The package for high pressure treatment according to claim 1, wherein said package body includes a portion composed of a rigid material and having an installation surface, and said irreversible pressure-sensitive marker comprises a print material formed on said installation surface of said package body by printing and containing microcapsules with pigment sealed therein, said microcapsules having a function of being destroyed and developing color upon being pressurized.

7. The package for high pressure treatment according to claim 5 or claim 6, wherein said package body includes a flanged container having a flange portion and also includes a top film bonded to said flange portion to maintain the articles accommodated within said flanged container in a sealed state, said flange portion being composed of a rigid material and having said installation surface.

8. The package for high pressure treatment according to claim 1, further comprising an irreversible temperature-sensitive marker provided on said package body and adapted to develop color at a heat treatment temperature.

9. The package for high pressure treatment according to claim 1, wherein said package body accommodates foods.

10. A method for treating foods at a high pressure, comprising:
accommodating foods within said package body of the package for high pressure treatment described in claim 1; and
subjecting the package for high pressure treatment to a high pressure treatment in the accommodated state of the foods.

11. A method for treating foods at a high pressure, comprising:
accommodating foods within said package body of the package for high pressure treatment described in claim 8; and
subjecting the package for high pressure treatment to both high pressure treatment and heat treatment in the accommodated state of the foods.
